# EUROPEAN PATENT APPLICATION

(11) **EP 1 360 930 A1**
(43) Date of publication of application: **12.11.2003**
(21) Application number: 03009968.3
(22) Date of filing: 30.04.2003
(51) Int. Cl.: A61B 5/024

(54) **pulse wave monitor**

(30) Priority: 09.05.2002 JP 2002134377
(71) Applicant: Omron Corporation, Kyoto-shi, Kyoto 600-8530 (JP)
(72) Inventor: Hashimoto, Masao, c/o Omron Corp., Shimogyo-ku, Kyoto-shi, Kyoto 600-8530 (JP); Itonaga, Kazunobu, c/o Omron Corp., Shimogyo-ku, Kyoto-shi, Kyoto 600-8530 (JP); Tanabe, Kazuhisa, c/o Omron Corp., Shimogyo-ku, Kyoto-shi, Kyoto 600-8530 (JP); Kitawaki, Tomoki, c/o Omron Corp., Shimogyo-ku, Kyoto-shi, Kyoto 600-8530 (JP); Kishimoto, Hiroshi, c/o Omron Corp., Shimogyo-ku, Kyoto-shi, Kyoto 600-8530 (JP)
(74) Representative: Kilian, Helmut, Dr.

(57) **Abstract**

A pulse wave monitor includes a sensor unit including a pulse wave sensor, and a base. The base of the pulse is mounted on a wrist of a subject. At this time, a finger or a detector is inserted through the pulse detecting hole provided on the base so as to detect an artery of the subject, and the base is fixed to the wrist of the subject by a belt so that the artery comes to a center of the base. The sensor unit is attached to the base by a clip. In this state, a position relationship between the sensor unit and the artery is detected. When a shift occurs, the sensor unit is slid so that mark is moved by a predetermined number of graduations, and the sensor unit is arranged in an optimal position so that a pulse wave of the subject is measured. As a result, it is possible to provide a pulse wave monitor capable of quickly performing positioning with high accuracy.

## Description

### BACKGROUND OF THE INVENTION

### Field of the Invention

The present invention relates to a pulse wave monitor and, more particularly, to a pulse wave monitor capable of quickly performing positioning with high accuracy.

### Description of the Related Art

When a pulse wave monitor measures a pulse wave of a subject, it measures a pushing pressure of a blood stream from a heart and a reflection pressure from a terminal. Since such a pulse wave is measured so that aging of a blood vessel of the subject can be known, the measurement of the pulse wave is very important to know a health condition of the subject.

In order to obtain the pulse wave of the subject accurately using the pulse wave monitor, it is essential to mount the sensor accurately on an artery of the subject.

Thus, U.S. Patent No. 5642733 discloses a pulse wave monitor having a structure that a base for positioning the sensor on the artery of the subject is preset on an organism, and a sensor housing is attached to the base.

Japanese Patent No. HB-2350 B2 (1996) or Japanese Utility Model Patent No. H4-28562Y (1992) discloses a pulse wave detector using a power generator such as a motor and moving a sensor housed in the unit to a direction directing to an artery of a subject so as to be capable of positioning the sensor automatically.

However, when the pulse wave monitor disclosed in U. S. Patent No. 5642733 is used to measure the pulse wave of the subject, a relative position between the base and the sensor housing cannot be finely adjusted, and when the sensor shifts from the artery of the subject after the attachment of the pulse wave monitor, the setting should be performed again starting from the positioning of the base. The positioning is troublesome and waste of time, thereby arising a problem in that convenience of a measurer is low and the subject is burdened.

In the case where the pulse wave detector disclosed in Japanese Patent No. H8-2350 B2 (1996) or Japanese Utility Model Patent No. H4-28562 Y (1992) is used to measure the pulse wave of the subject, when the sensor is mounted on the artery of the subject detected by palpation or the like, the sensor cannot be visually inspected, and thus a possibility in that a center of the sensor shifts greatly from the artery at the stage of mounting the sensor is strong, thereby arising a problem in that the positioning accuracy is not good at the stage of setting the sensor. Since after the sensor is mounted, the power generator such as the motor is used to automatically position the sensor, there arises a problem in that a cost is high and the pulse wave detector is not adequate to miniaturization.

### SUMMARY OF THE INVENTION

The present invention is devised in order to solve these problems. An object of the present invention is to provide a pulse wave monitor capable of quickly performing positioning with high accuracy, and another object thereof is to provide a pulse wave monitor which is suitable for miniaturization at low cost.

In order to achieve the above objects, according to an aspect of the present invention, a pulse wave monitor measuring a pulse wave of a subject includes: a sensor unit including a sensor detecting the pulse wave; a base configured to be mounted on a portion of the subject and to engage with the sensor unit, the base including a positioning portion through which an artery of the subject is detected; and a slide mechanism for sliding the sensor unit relative to the base when the base is engaged with the sensor unit.

Desirably, the positioning portion of the base has an opening through which the artery of the subject is detected.

Desirably, the positioning portion of the base has a hole through which the artery of the subject is detected.

Desirably, the positioning portion of the base has a cut away portion through which the artery of the subject is detected.

Desirably, the pulse wave monitor further includes a display providing information about a positioning of the sensor with respect to the artery.

Desirably, an array of markings is formed on the sensor unit, the base or the slide mechanism so that the array is positioned perpendicular to the direction of the array when the base is mounted on the portion of the subject and is engaged with the sensor unit.

Desirably, the pulse wave monitor further includes a subject fixture including a first attaching portion and a second attaching portion, wherein a first portion of the base is attached to the first attaching portion and a second portion of the base is adjustably attached to the second attaching portion.

Desirably, the subject fixture further includes a pulling mechanism that applies a tension to the second portion of the base.

According to another aspect of the present invention, a pulse wave monitor measuring a pulse wave of a subject includes: a base configured to be mounted on a portion of the subject; a sensor unit including a sensor detecting the pulse wave; a part for coarsely positioning the base on the portion of the subject; and a part for finely positioning the sensor unit with respect to the portion of the subject.

Desirably, the pulse wave monitor further includes a subject fixture including a first attaching portion and a second attaching portion, wherein a first portion of the base is attached to the first attaching portion and a second portion of the base is adjustably attached to the second attaching portion.

Desirably, the subject fixture further includes a pulling mechanism that applies a tension to the second portion of the base.

### BRIEF DESCRIPTION OF THE DRAWINGS

Fig. 1 illustrates a specific example of an appearance of a pulse wave monitor according to an embodiment;
Figs. 2A and 2B illustrate specific examples of appearances of a sensor unit;
Fig. 3 is a functional block diagram showing a structure of the sensor unit;
Fig. 4 illustrates an outline of a position relationship between a sensor and an artery of a subject;
Fig. 5 illustrates a structure of a base;
Fig. 6 illustrates an attaching outline of the sensor unit and the base;
Figs. 7A and 7B illustrate a portion where the sensor unit and the base are fixed;
Fig. 8 illustrates another specific example of the sensor unit and the base;
Fig. 9 illustrates still another specific example of the sensor unit and the base;
Fig. 10 illustrates yet another specific example of the sensor unit and the base;
Fig. 11 illustrates yet another specific example of the sensor unit and the base;
Fig. 12 illustrates yet another specific example of the sensor unit and the base;
Fig. 13 is a flowchart showing a positioning process for the pulse wave monitor according to the embodiment;
Fig. 14 illustrates a specific example of a display provided on the sensor unit of the pulse wave monitor;
Fig. 15 shows a first specific example of a screen displayed by the display;
Fig. 16 shows a second specific example of a screen displayed by the display;
Fig. 17 shows a third specific example of a screen displayed by the display;
Fig. 18 illustrates a specific example of the display including LED provided on the sensor unit of the pulse wave monitor;
Fig. 19 shows a specific example of a measured result displayed on the display;
Fig. 20 illustrates a specific example of a structure in the case where the pulse wave monitor according to the embodiment includes a fixture;
Fig. 21 illustrates a state where the pulse wave monitor according to the embodiment including the fixture measures a pulse wave of the subject.
Fig. 22 illustrates an attaching portion of the fixture;
Fig. 23 is a schematic sectional view taken along plane A in Fig. 22;
Fig. 24 illustrates a fixing portion of the fixture; and
Fig. 25 illustrates another specific example of the sensor unit and the base.

### DETAILED DESCRIPTION OF THE PREFERRED EMBODIMENTS

Hereinafter, embodiments of the present invention will be described with reference to the drawings. In the following description, the same parts and same components are denoted by the same reference numerals . Those names and functions are also same; therefore, those specific descriptions will not be repeated.

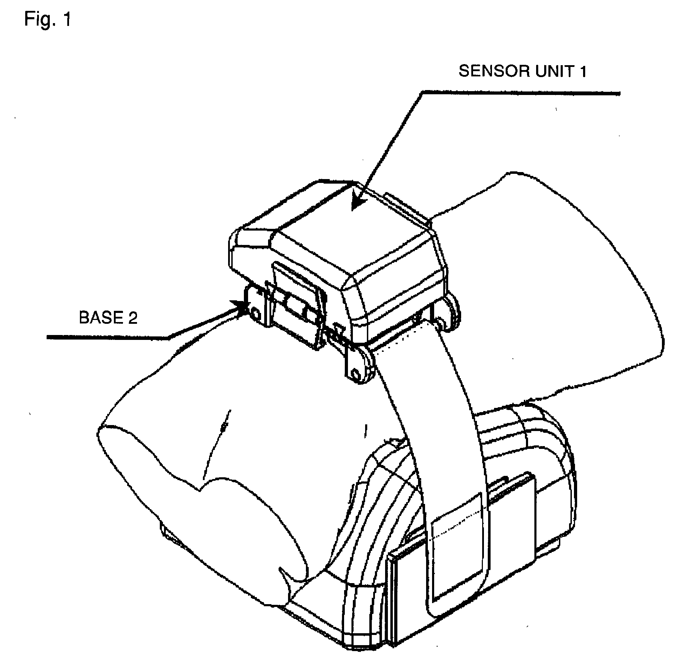

Fig. 1 illustrates a specific example of an appearance of a pulse wave monitor according to an embodiment.

With reference to Fig. 1, a pulse wave monitor according to the embodiment includes: a sensor unit 1 having a pressure sensitive element for pressing a portion of a subject on which a pulse wave is measured so as to detect the pulse wave of the subject; and a base 2 for positioning the sensor unit 1. The pulse wave monitor in the embodiment measures a pulse wave of a subject on a wrist of the subject. Therefore, Fig. 1 shows a state where the pulse wave monitor is mounted on the wrist of the subject.

Description will be given of each of the sensor unit 1 and base 2 included in the pulse wave monitor according to the embodiment, and description will be also given of the use of the pulse wave monitor.

### (1) Sensor Unit 1

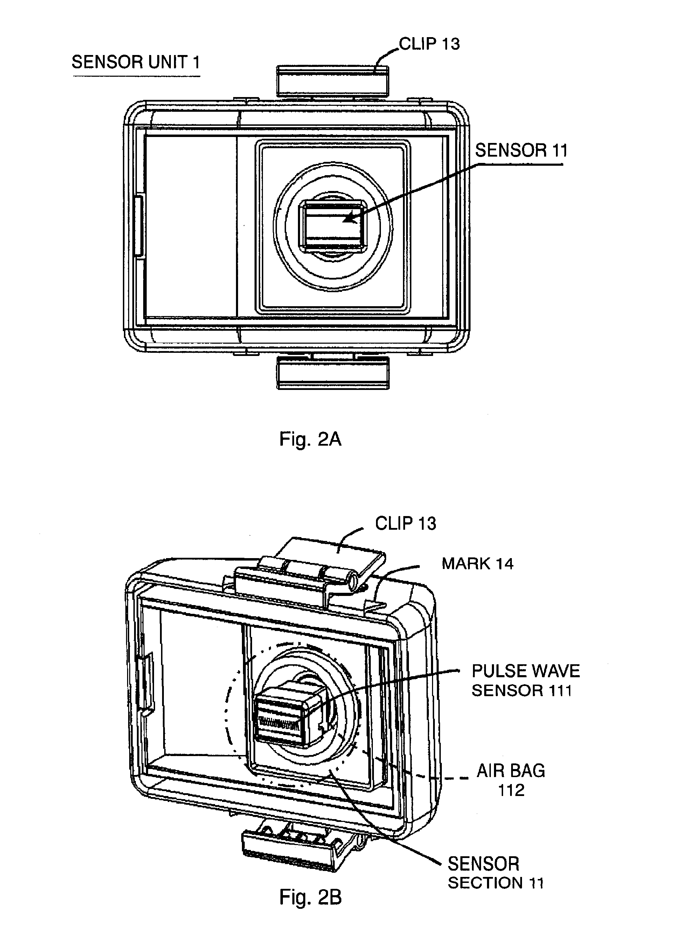

Figs. 2A and 2B illustrate specific examples of appearances of a sensor unit 1. Fig. 2A illustrates a state where the sensor unit 1 is viewed from below. Fig. 2B illustrates a state where the sensor unit 1 is viewed from a lower direction to an obliquely up direction.

With reference to Figs. 2A and 2B, the sensor unit 1 includes: a sensor 11; a clip 13 for attaching the sensor unit 1 to the base 2; and a mark 14 on a surface including a side which is brought into contact with the base 2 when the sensor unit 1 is attached to the base 2. The sensor 11 includes: a pulse wave sensor 111 for detecting the pulse wave of the subject; an air bag 112 for pressing the pulse wave sensor 111 against an organism on a rear side of the pule wave sensor 111; and a display 113 (not shown).

The pulse wave sensor 111 includes the pressure sensitive element, and detects the pulse wave of the subject by means of a pressure applied from the artery of the subject. One pulse wave sensor 111 may be provided on a surface of the sensor 11, which is brought into contact with the wrist of the subject (sensor surface), or a plurality of them may be provided thereon in parallel.

The display 113, not shown in Figs. 2A and 2B, is provided on a position which can be seen from a measurer such as an upper surface of the sensor unit 1, and notifies the measurer of whether the pulse wave monitor measures the pulse wave in a lightening manner or the like.

When the pulse wave monitor measures the pulse wave of the subject, air is injected into the air bag 112 included in the sensor 11, and the pulse wave sensor 111 is pressed against the artery of the subject. The pulse wave sensor 111 detects a pulse pressure of the subject. The position relationship between the sensor unit 1 and the artery of the subject at the time of the measurement will be described later.

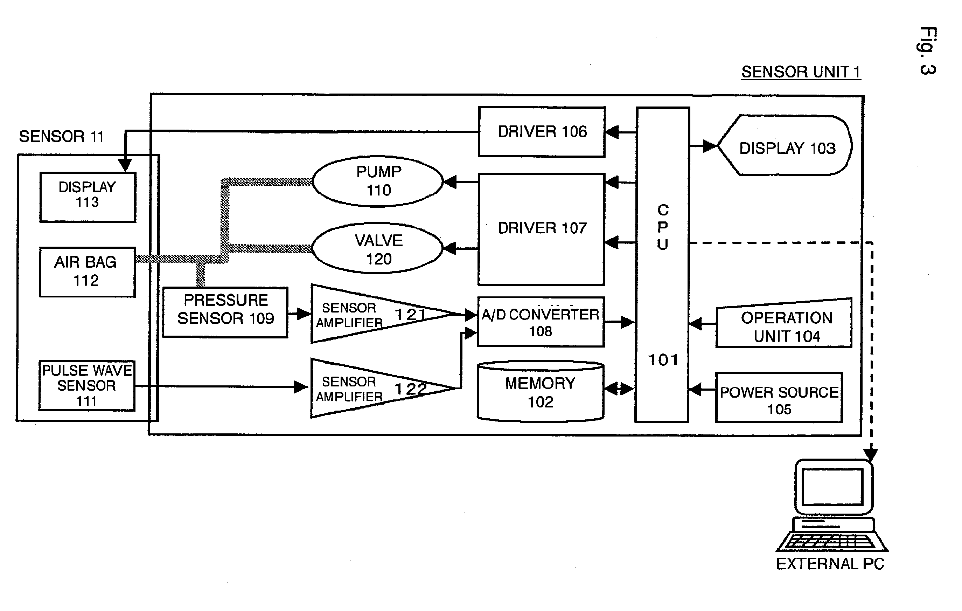

Fig. 3 is a functional block diagram showing a structure of the sensor unit 1.

With reference to Fig. 3, the sensor unit 1 includes: a CPU (Central Processing Unit) 101 for controlling the entire sensor unit 1; a memory 102 for storing a program and the like to be executed in the CPU 101; and a power source 105 for supplying a power to the sensor unit 1.

The sensor unit 1 accepts an instruction of the measurer through an operation unit 104 formed of an operating button shown in Fig. 3 so as to measure the pule wave.

A pressure sensor 109 included in the sensor unit 1 detects a pressure of the air bag 112 of the sensor 11. The pressure of the air bag 112 is a pushing pressure generated when the pulse wave sensor 111 is pressed onto the artery, and is controlled by the CPU 101. A detected analog pressure value of the air bag 112 is transmitted to an A/D (Analog to Digital) converter 108 via a sensor amplifier 121 so as to be converted into a digital value, and the digital value is transmitted to the CPU 101. The CPU 101 controls a pump 110 and a valve 120 via a driver 107 so as to control a quantity of the air to be injected into the air bag 112.

Similarly, the analog pulse pressure value of the subject detected by the pulse wave sensor 111 of the sensor 11 is transmitted to the A/D converter 108 via a sensor amplifier 122 so as to be converted into a digital value, and the digital value is transmitted to the CPU 101.

A signal from the CPU 101 is transmitted to a display 113 via a driver 106.

The position relationship between the sensor 11 and the artery of the subject and information about the measured pulse wave of the subject are outputted from the CPU 101 to a display portion 103 formed of a display and the like so as to be displayed. The display will be described later. When the sensor unit 1 has a connecting function, the information may be outputted to a connected external personal computer (hereinafter, referred to as PC).

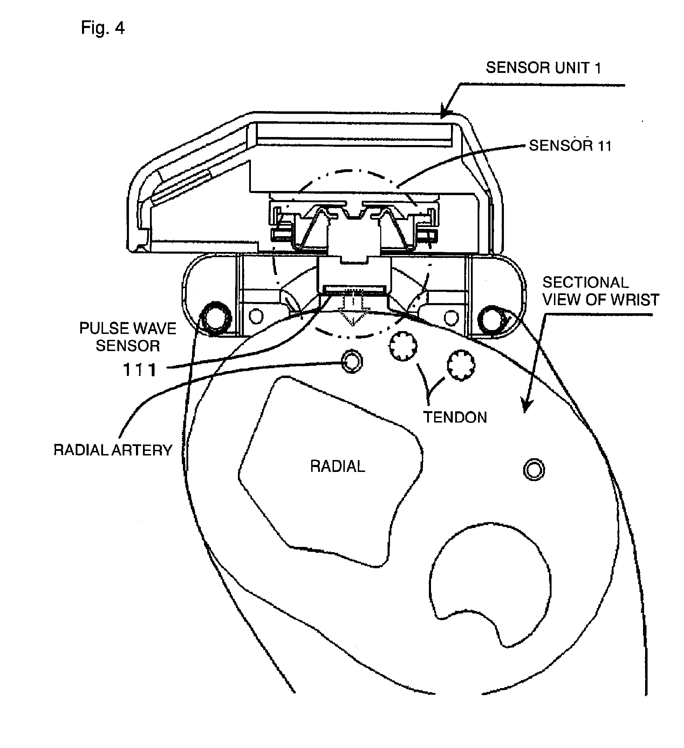

Fig. 4 illustrates theoutlineof the position relationship between the sensor 11 and the artery of the subject.

As shown in Fig. 4, when the pulse wave monitor measures the pulse wave of the subject, it is desirable to mount the sensor 11 just on the artery of the subject. More specifically, when the pulse wave is measured on the wrist of the subject, it is desirable to mount the sensor 11 just on a radial artery of the subject. As described above, when the air is injected into the air bag 112 of the sensor 11, the pulse wave sensor 111 is pressed just on the artery of the subject so as to detect a pressure of the artery.

The structure of the sensor unit 1 is not limited to the above structure, and a structure may be adopted such that a part of the sensor unit 1 is separated. This case has an advantage such that a size of the portion to be attached to the subject can be small.

### (2) Base 2

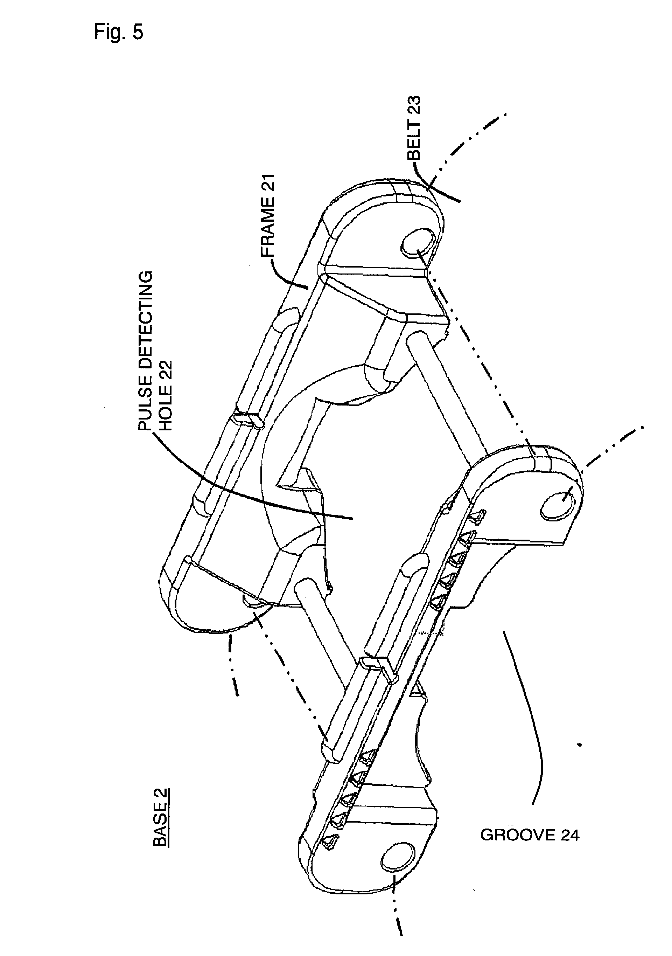

Fig. 5 illustrates a structure of the base 2 included in the pulse wave monitor shown in Fig. 1.

With reference to Fig. 5, the base 2 includes a frame 21 on which the sensor unit 1 is mounted; and a pulse detecting hole 22 through which the pulse of the subject is detected. The base 2 further includes a belt 23 for fixing the base 2 to a pulse wave detecting portion of the subject, and the belt 23 is wound to a direction which intersects the artery of the subject, thereby fixing the base 2 to the pulse wave detecting portion of the subject.

The pulse detecting hole 22 is an opening which is provided on a position on the frame 21 where the pulse of the subject can be detected. In other words, this is the opening which is provided on the frame 21 of the base 2 in an up direction (in Fig. 5, a vertical direction). The shape of the pulse detecting hole 22 is a square in Fig. 5, but the shape is not limited. Its size may be such that the measurer can insert at least one finger into the hole in order to palpate a pulse of the subject, and it may have a hole-like shape. When a sensor such as a Doppler blood-flowmetry is used to detect the pulse of the subject, the sensor may have a size such that it touches the subject. In this case, the base 2 may have a setting unit of the sensor, not shown in Fig. 5.

### (3) Attachment between Sensor Unit 1 and Base 2

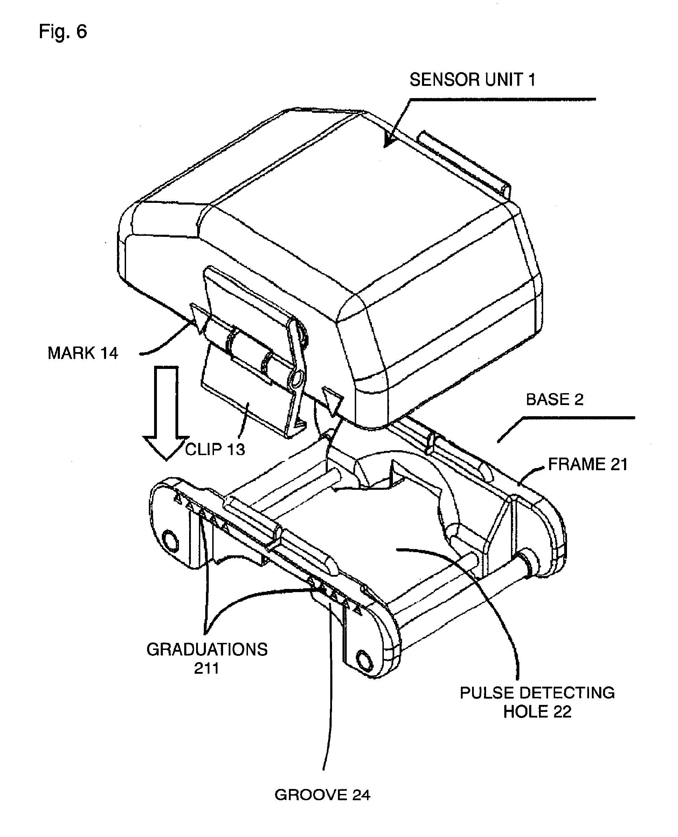

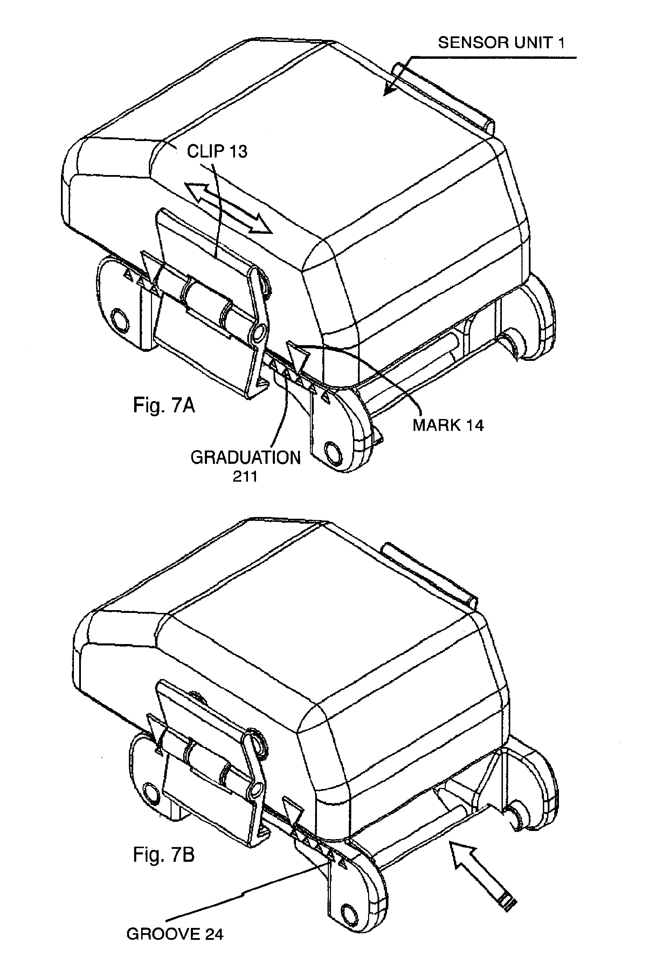

When the pulse wave monitor measures the pulse wave of the subject, the sensor unit 1 and the base 2 are attached. Fig. 6 illustrates an outline of the attachment between the sensor unit 1 and the base 2. Figs. 7A and 7B illustrate a portion where the sensor unit 1 and the base 2 are fixed.

With reference to Figs. 6, 7A and 7B, the frame 21 and the sensor unit 1 are fixed in such a manner that a pawl provided on the clip 13 of the sensor unit 1 is engaged with a groove 24 provided on the frame 21. A side surface of the frame 21 which intersects the artery of the subject has graduations 211 for determining a position of the sensor unit 1.

Aperson pinches the sensor unit 1 using fingers and applies a force to it, thereby sliding the sensor unit 1 on the frame 21 along the groove 24 to a direction which intersects the artery of the subject (direction shown by an arrow in Figs. 7A and 7B). Fig. 7B shows a state where the sensor unit 1 shown in Fig. 7A is slid to a left direction in the figure. A force for sliding the sensor unit 1 may be a driving force of a compact motor or the like. As to a method of sliding the sensor unit 1, a fixing force of the clip 13 is loosened by a finger and simultaneously the sensor unit 1 may be slid according to its structure. A lever, a button or the like, not shown, is operated, so that the sensor unit 1 may be slid mechanically by using a gear or the like or slid along a rail (not shown), or slid by the driving force of the motor or the like.

The marks 14 provided on the sensor unit 1 and graduations 211 provided on the frame 21 of the base 2 become a guidepost of a sliding amount when the sensor unit 1 is slid. The measurer visually checks the graduations 211 and simultaneously slides the mark 14 of the sensor unit 1 by a predetermined number of graduations, so as to be capable of accurately positioning the sensor unit 1. The positioning of the sensor unit 1 will be described later. When the sensor unit 1 or the base 2 has a function of notifying of the sliding of the sensor unit 1 by one graduation 211 using sound, light, vibration or the like, the measurer can check the information and simultaneously slide the sensor unit 1.

In view of convenience of measurer, it is desirable to provide the mark 14 and graduations 211 on left and right sides with respect to the centers of the sensor unit 1 and the frame 21. This is because if, for example, the measurer is right-handed, in most, the sensor unit 1 is frequently slid by the right hand. In this case, the mark 14 and the graduations 211 which are provided on the right sides with respect to the centers of the sensor unit 1 and the frame 21 are hidden by the measure's right hand, and thus it is difficult to check the mark 14 and the graduations 211 and simultaneously slide the sensor unit 1. When the measurer is right-handed, it is desirable to provide the mark 14 and the graduations 211 on the left sides with respect to the centers of the sensor unit 1 and the frame unit 21. When the measurer is left-handed, it is desirable to provide the mark 14 and the graduations 211 on the right sides with respect to the centers of the sensor unit 1 and the frame 21. The graduations 211 may be printed on the frame 21. The mark 14 of the sensor unit 1 and the graduations 211 of the frame 21 may form a pair of uneven shapes. Specifically, in the latter case, the mark 14 of the sensor unit 1 and the graduations 211 of the frame 21 are engaged with each other on every one graduation, and the sensor unit 1 is slid by every one graduation. When a plurality of the pulse wave sensors 111 of the sensor unit 1 are provided on the sensor surface of the sensor 11 as described above, it is desirable that the graduations 211 of the frame 21 has a graduation width according to a setting pitch of the pulse wave sensors 111. The width of the graduations 211 is equal with a maximum moving amount of the sensor unit 1 and the gradation widths are uniform so as to become a guidepost at the time of the movement of the sensor unit 1, thereby enabling the more accurate positioning.

### (4) Another Specific Examples of Sensor unit 1 and Base 2

The above-described structures of the sensor unit 1 and the base 2 are one specific example. The structure of the sensor unit 1 and the base 2, therefore, is not limited to the above structures, and may have another structures. Another specific examples will be described below.

Specifically, the mark 14 of the sensor unit 1 and the graduations 211 of the frame 21 may be replaced by each other. That is, the graduations 211 may be provided on the sensor unit 1, and the mark 14 may be provided on the frame 21. The clip 13 may be provided on the frame 21 of the base 2. The method of fixing the sensor unit 1 and the base 2 is not limited to the method of engaging the pawl of the clip 13 with the groove 24 of the base 2 as shown in Figs. 6, 7A and 7B.

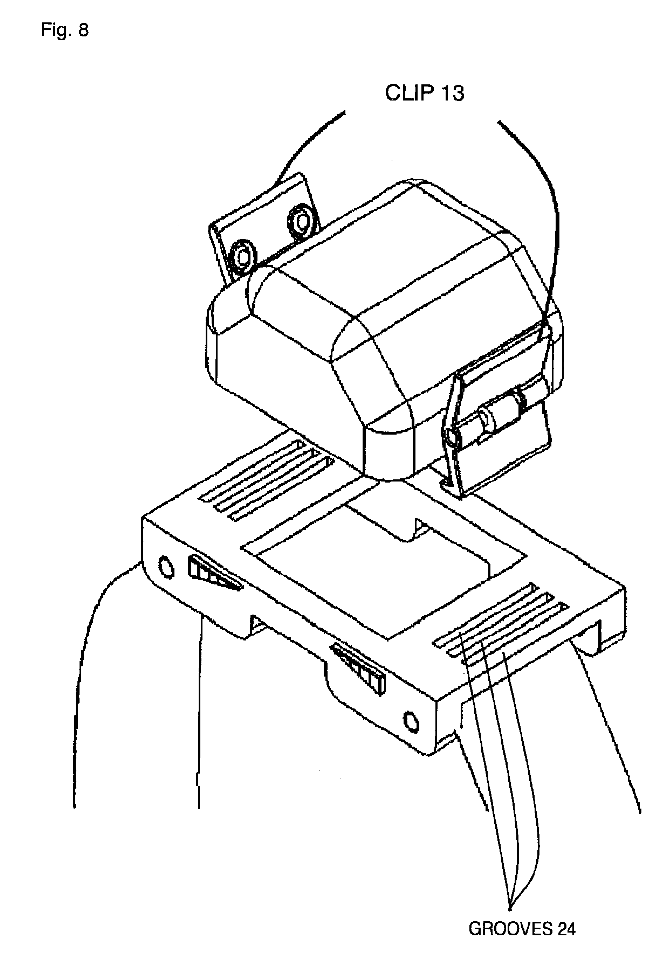

As shown in Fig. 8, the clips 13 may be provided on side surfaces parallel with the artery of the subject. In this case, a plurality of grooves 24 are provide at positions of the base 2 corresponding to the clips 13 on both sides of the sensor unit 1 in a direction orthogonal to the artery of the subject.

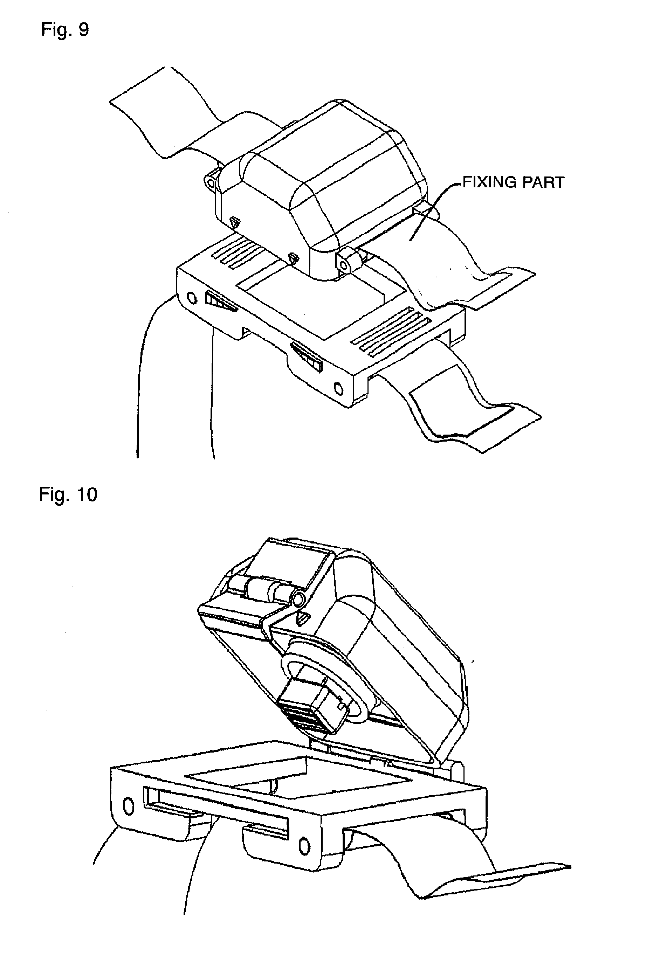

As shown in Fig. 9, fixing parts such as surface fasteners may be provided on the sensor unit 1 instead of the clips 13.

As shown in Fig. 10, the structure is not such that the sensor unit 1 and the base 2 are separated from each other completely, but the structure may be adopted such that the sensor unit 1, which intersects the artery of the subject, is coupled with the base 2 via a hinge at its one edge of a side surface in the sliding direction, the sensor unit 1 is slid. One edge of the side surface parallel with the artery of the subject may be coupled with the base 2 via the hinge. The structure may be adopted such that the sensor unit 1 and the base 2 are fixedly integrated as one unit, and only the sensor 11 of the sensor unit 1 is slid. In this case, the pulse detecting hole 22 is provided on the sensor unit 1, and palpation or the like is performed so that the base 2 can be mounted on the artery of the subject. As described above, in the case where the sensor unit 1 and the base 2 are coupled via the hinge, or in the case where the sensor unit 1 and the base 2 are completely integrated as one unit, when the sensor unit 1 and the base 2 are opened, it is desirable that the sensor 11 automatically returns to a default position (center or the like) of the sensor unit 1.

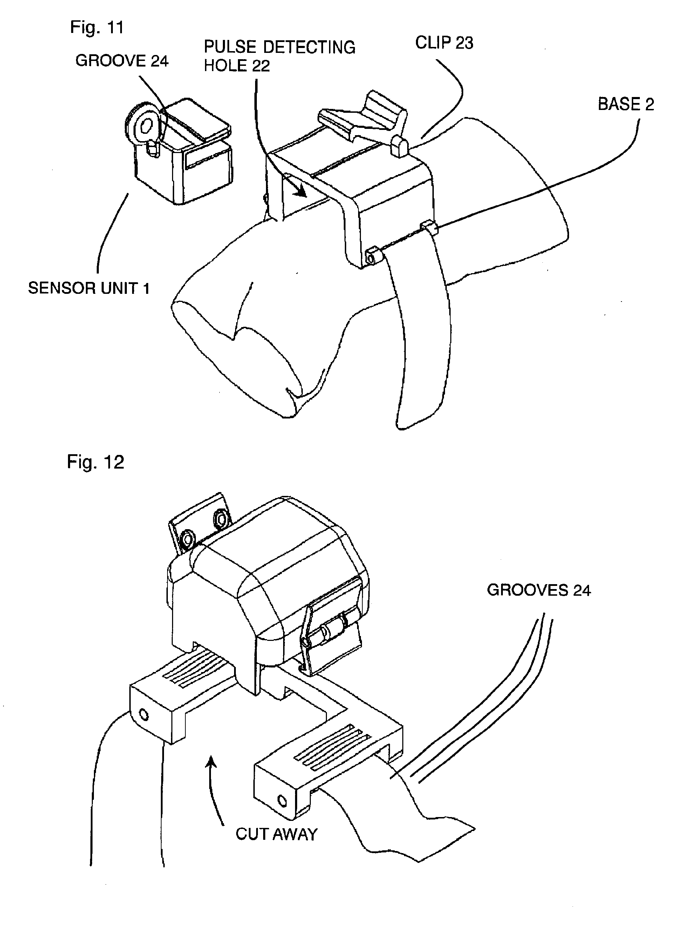

The pulse detecting hole 22 provided on the base 2, as shown in Fig. 5, is not limited to a hole in the just up direction of the base 2. That is, as shown in Fig. 11, the pulse detecting hole 22 may be provided on the side surface of the base 2 intersecting the artery of the subject in a direction parallel to the artery of the subject. In this case, the base 2 and the sensor unit 1 are in a state where the pawl of the clip 13 provided on the base 2 is engaged with the groove 24 provided on the upper portion of the sensor unit 1, so that the pawl of the clip 13 can slide in the groove 24. As a result, the positions of the sensor unit 1 and the base 2 can be moved to arbitrary positions and be fixed there.

As shown in Fig. 12, a cut away may be provided on the side surface of the base 2 intersecting the artery of the subject. When the cut away shown in Fig. 12 is provided on the base 2, the measurer can touch a wide area of the subject with a finger without being disturbed by the frame 21, thereby facilitating the palpation of the artery of the subject.

The pulse detecting hole 22 provided on the base is not a through hole, but it may be provided with a film of such thickness and material that the detection of the pulse of the subject is not obstructed. Specifically, a film or the like made of silicon rubber with a thickness of not more than 0.2 mm is desirable. When the pulse detecting hole has such a film, the sensor 11 provided on the sensor unit 1 is not directly brought into contact with the subject, so that the surface of the sensor 11 can be protected. When the pulse wave monitor is used for a plurality of subjects, sanitary conditions can be managed.

The pulse detecting hole 22 provided on he base 2 may be provided with a transparent film or plate pre-marked with "X" or the like on its center. The measurer aligns the mark with the artery of the subject detected by palpation or the like so as to be capable of mounting the base 2. Therefore, the base 2 can be mounted just on the artery of the subject accurately.

The sensor 11 of the sensor unit 1 further has a height sensor for detecting a height to be measured besides the pulse wave sensor 111 for detecting the pulse wave of the subject, and the pulse wave monitor may have a height adjusting mechanism.

When the pulse wave is generally measured, it is desirable that a portion to be measured is positioned at the same height as the heart of the subject. As a difference of elevation from the heart is larger, a possibility in that the measurement is influence by a difference in a water pressure of the blood becomes stronger. Therefore, the height sensor may detect a difference of elevation from the heart of the subject, and the height sensor may measure the height of the portion to be measured actually by pre-inputting a height or the like of the subject so as to detect a difference of elevation from the heart of the subject. When the portion to be measured is the wrist, the sensor may be an angle sensor using an angle as a parameter. In this case, a length and an angle of an arm are obtained so that a difference of elevation from the heart of the subject can be detected.

### (5) Positioning of Pulse Wave Monitor

When the pulse wave monitor measures the pulse wave, it is necessary to perform the positioning prior to the measurement of the pulse wave. That is, it is necessary to position the sensor unit 1 so that the unit sensor 1 is mounted in a range where the pulse wave of the artery of the subject can be detected. The position of the sensor unit 1 which is within the range where the pulse wave of the artery of the subject can be detected is simply referred to as a detecting position.

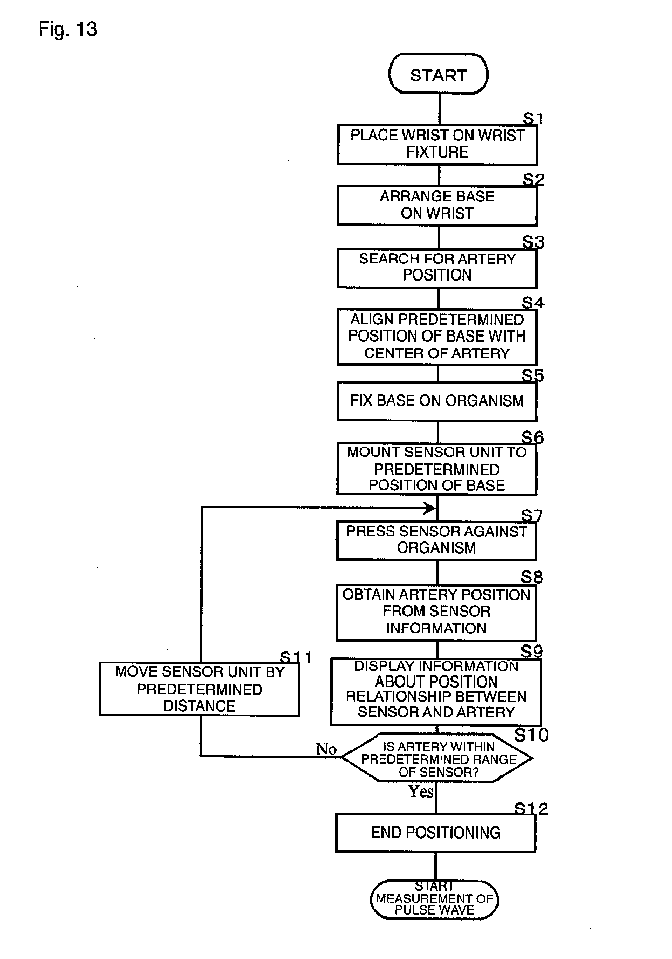

Fig. 13 is a flowchart showing the positioning process for the pulse wave monitor according to the embodiment. The embodiment will describe the case where the pulse wave is measured at the artery of the subject's wrist.

At first, the wrist of the subject is placed on a wrist fixture 3 (S1). The wrist fixture 3 will be described later.

The base 2 of the pulse wave monitor is mounted on the wrist of the subject (S2).

The pulse of the subject is detected through the pulse detecting hole 2 of the base 2 (S3). As described above, the measurer may insert a finger through the pulse detecting hole 22 and palpate the subject so as to detect the pulse, or may insert an artery detecting device such as a Doppler blood-flowmetry through the pulse detecting hole 22 so as to detect the pulse.

The base 2 is positioned so that a position of the detected artery comes to the center of the base 2 (S4). For convenience of a following description, the positioning of the base 2 is referred to as first positioning.

The positioned base 2 is fixed onto the wrist of the subject (55). The fixing method as well as the wrist fixture will be described below.

The sensor unit 1 is attached to a predetermined position on the fixed base 2 (S6). Herein, it is desirable that the sensor unit 1 is attached to the approximately center of the base 2.

In this state, the pulse wave sensor 111 of the sensor unit 1 is pressed against the wrist of the subject and the pulse wave of the subject is temporarily measured (S7), so that a position of the artery of the subject is obtained from sensor information (S8).

At this time, the position relationship between the sensor 11 and the artery is displayed on the display (S9), so that the position of the artery of the subject can be obtained accurately, and the sensor unit 1 can be mounted on the detected position. The display will be described below.

When the artery of the subject is within the detectable range of the pulse wave sensor 111, that is, when the sensor unit 1 is set on the detecting position (YES in S10), the positioning process is ended (S12), and the measurement of the pulse wave of the subject is started.

When the artery of the subject is not within the detectable range of the pulse wave sensor 111, that is, when the sensor unit 1 is not set on the detecting position (NO in S10), the sensor unit 1 is slid by predetermined graduations (S11), and the process is again started from the step S7 for temporarily measuring the pulse wave of the subject so that the position of the sensor unit 1 determined. For convenience of the description, the positioning of the sensor unit 1 is referred to as second positioning.

The positioning process of the pulse wave monitor is ended, and the measurement of the pulse wave of the subject is started.

At step S11, when only the sensor 11 of the sensor unit 1 is slid as described in another specific example of the sensor unit 1 and the base 2, the sensor 11 is slid so that the second positioning may be performed.

The positioning of the pulse wave monitor in the embodiment is characterized, as described above, in that two-stage positioning including the first positioning and the second positioning is performed.

When the first positioning is performed, since the base 2 has the pulse detecting hole 22, the palpation or the like is conducted and the pulse of the subject is detected, so that the base 2 can be set with high accuracy. That is, when the sensor unit 1 is attached to the base 2 at the time when the first positioning is completed, the pulse wave of the subject can be measured in approximately that state. Therefore, the second positioning means fine adjustment, and when the positioning is performed by using the pulse wave monitor of the embodiment, the second positioning is not originally presupposed.

When the second positioning is performed, as described at step S9, the position relationship between the sensor 11 and the artery is displayed on the display, so that the accuracy of the fine adjustment can be heightened. It is therefore desirable that the pulse wave monitor of the embodiment has the display.

As described at step S1, the wrist of the subject is fixed to the wrist fixture 3, so that the positioning and the measurement of the pulse wave can be performed adequately. In order that the pulse wave sensor 111 of the sensor unit 1 measures the pulse wave of the subject, it is desirable to pressure the wrist of the subject with a suitable pressure. As described above, when the sensor unit 1 is slid in the first positioning, it is desirable that the base 2 pressures the wrist of the subject with suitable pressure so that the base 2 is not moved cooperatively. It is therefore desirable, that the pulse wave monitor of the embodiment has the wrist fixture 3.

The display and the wrist fixture 3 of the pulse wave monitor will be described below.

### (6) Display

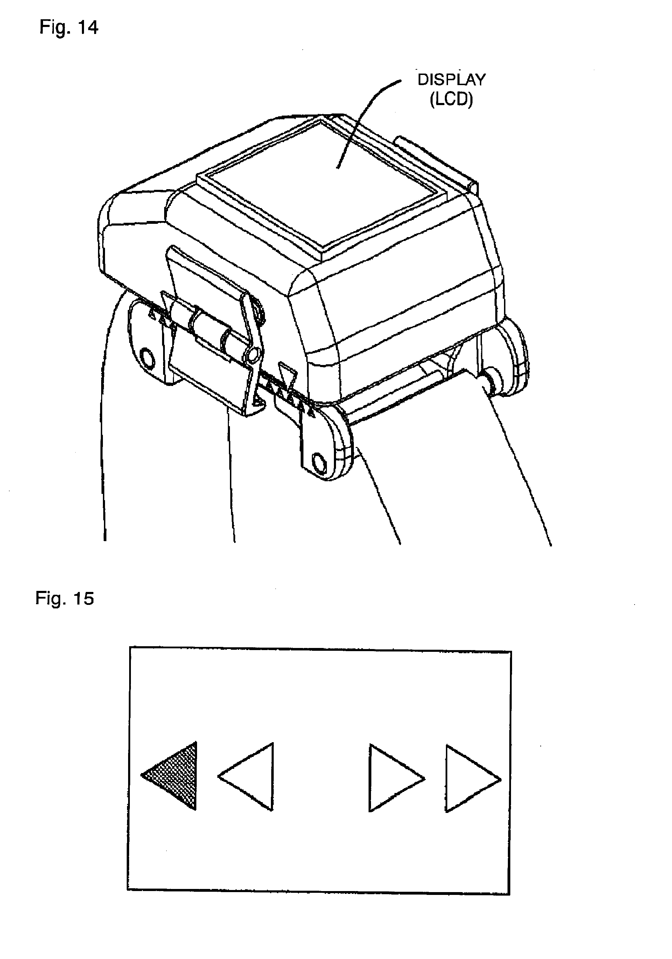

Fig. 14 illustrates a specific example of the display provided on the sensor unit 1 of the pulse wave monitor.

With reference to Fig. 14, the display is formed of LCD (Liquid Crystal Display) and is provided at a position on the upper portion of the sensor unit 1 which is easily seen by the measurer.

Figs. 15 to 17 show specific examples of a screen displayed on the display.

In a first specific example shown in Fig. 15, a direction to which the sensor unit 1 shifts from the artery of the subject, or a direction to which the sensor unit 1 is moved is displayed on the display. An amount of the shift from the artery or the moving amount may be also displayed.

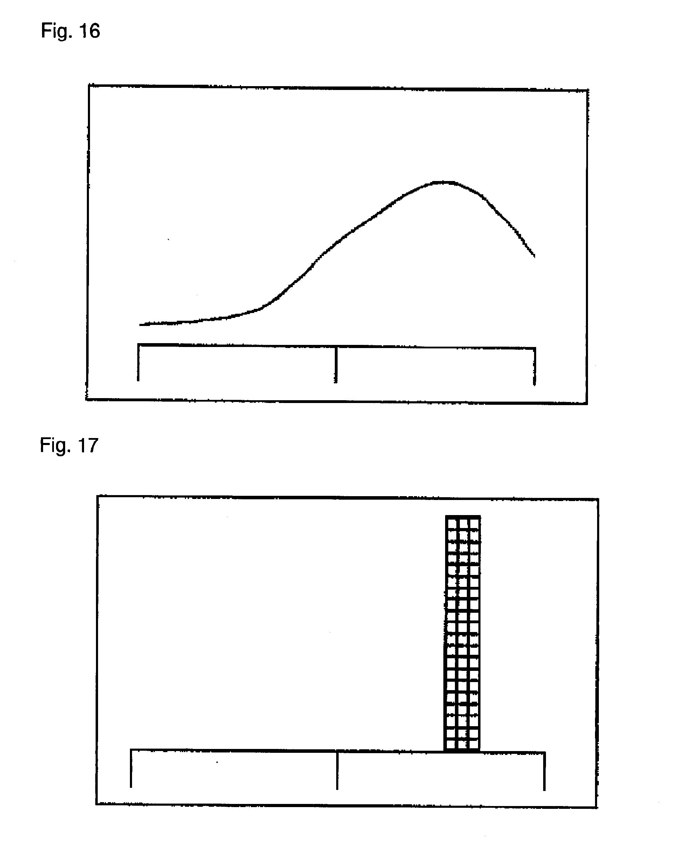

In a second specific example shown in Fig. 16, a tonogram showing a distribution of the pulse pressure detected by the pule wave sensor 111 of the sensor unit 1 is displayed. When the sensor unit 1 is mounted on the detecting position, a peak value of the pulse wave monitor is shown on a center of the tonogram. When, therefore, the peak value deviates to right or left, the measurer refers to the display screen shown in Fig. 16 so as to move the sensor unit 1 to a direction where the peak value is moved to the center.

In a third specific example shown in Fig. 17, the center position of the artery with respect to the pulse wave sensor 111 of the sensor unit 1 or a width at which the artery exists is displayed. With reference to the display screen shown in Fig. 17, the measurer moves the sensor unit 1 to the center position of the artery or a direction where the width of the artery is moved to its center similarly to the tonogram.

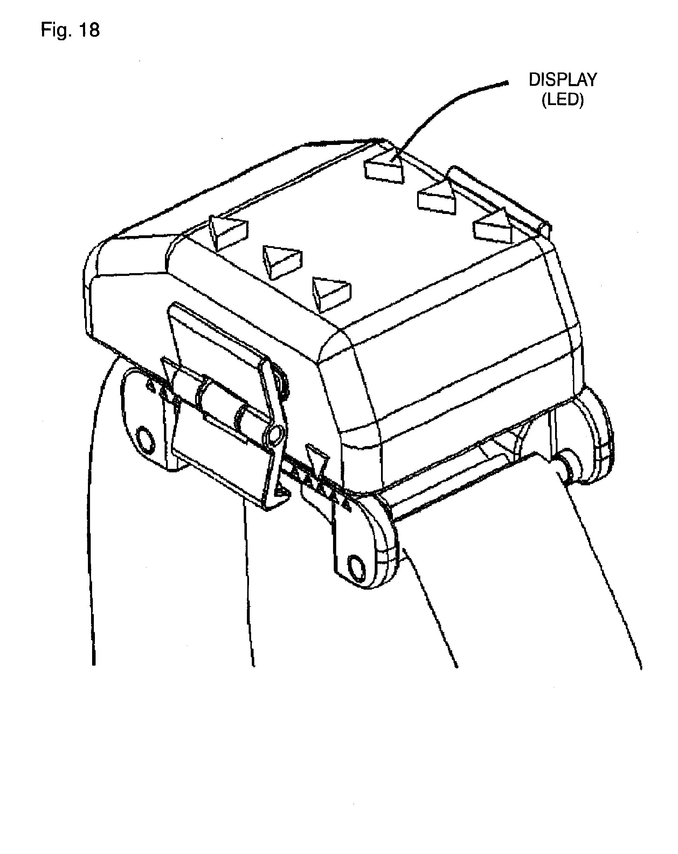

As shown in Fig. 15, when the shifting direction of the sensor unit 1 from the artery of the subject or the moving direction of the sensor unit 1 are displayed on the display, the display is not formed of LCD but of LED (Light Emitting Diode). Fig. 18 shows a specific example of the display formed of the LED provided on the sensor unit 1 of the pulse wave monitor. As shown in Fig. 18, the display formed of the LED has a triangular or an arrow shape, and the display showing a predetermined direction emits light so as to show the shift direction of the sensor unit 1 from the artery of the subject or the moving direction of the sensor unit 1. When a plurality of displays having the triangular shape or the arrow shape are provided, a shift amount from the artery or a moving amount can be also displayed according to a number of the lighting displays.

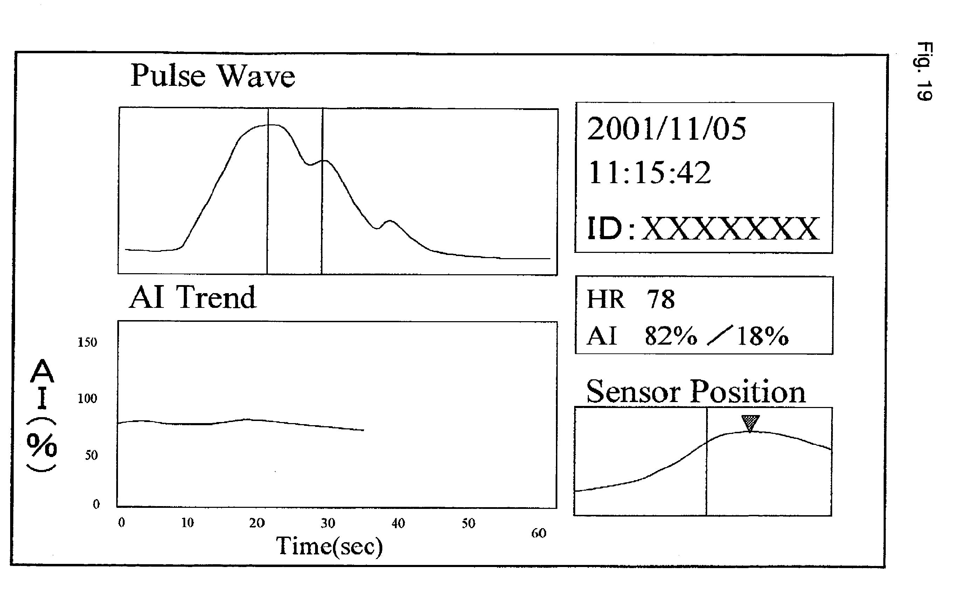

As shown in Figs. 15 to 17, the displays formed of LCDs are provided on the sensor unit 1, the pulse wave measured by the pulse wave monitor can be displayed. That is, Fig. 19 shows a specific example of the measured result displayed on the displays.

The displays are not limited to the case where they are provided on the sensor unit 1, and may be provided on the base 2. As shown in the functional block diagram of Fig. 3, when an external PC or the like can be connected to the pulse wave monitor, display may be performed on the connected external PC or the like.

### (7) Wrist fixture 3

As described above, in the sensor unit 1, when air is injected into the air bag 12, the pulse wave sensor 111 pressurized against the artery of the subject detects the pulse pressure of the subject so as to detect the pulse wave of the subject. Therefore, the base 2 attached to the sensor unit 1 lifts up from the wrist of the subject due to reaction against the injection of the air into the air bag 12 and the pressurizing, and thus the pulse wave sensor 111 is not occasionally pressurized against the wrist of the subject properly. In order to avoid this situation, it is necessary that the base 2 is fixed to the wrist of the subject with a suitable pressure. It is therefore desirable that the pulse wave monitor of the embodiment has the wrist fixture 3 (hereinafter, simply referred to as the fixture 3).

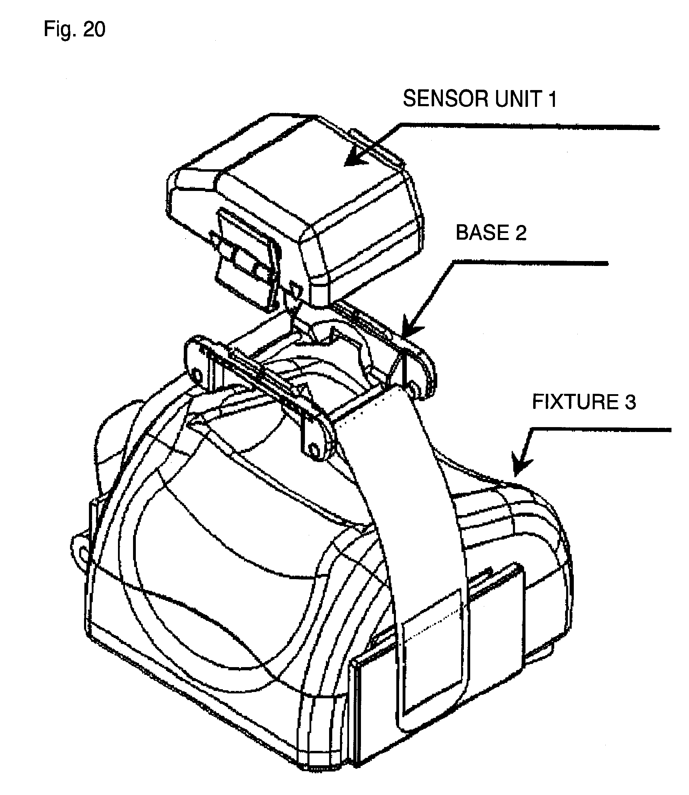

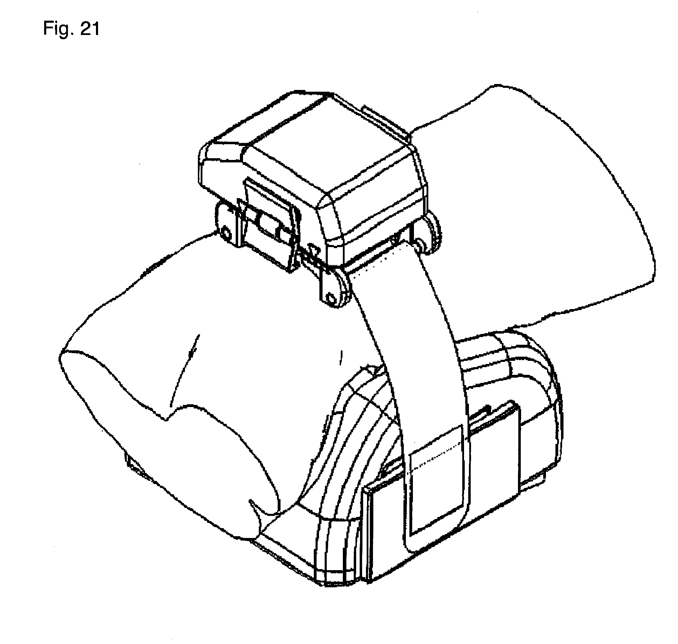

Fig. 20 illustrates a specific example of the structure that the pulse wave monitor of the embodiment has the fixture 3. Fig. 21 illustrates a state where the pulse wave monitor of the embodiment having the fixture 3 measures the pulse wave of the subject.

With reference to Figs. 20 and 21, the fixture 3 fixes the base 2 to the wrist of the subject with an appropriate pressure as described above, it has a shape that it is fitted to the wrist. In view of convenience of the subject and measurer, the fixture desirably has a shape such that it is fitted to both left and right wrists so that the measurement can be performed on both the left and right wrists of the subject. The fixture 3 desirably has a shape such that it is fitted to a wrist which is placed from any directions so that a direction with respect to the subject is not limited. The fixture 3 may be made of a material such that when the wrist of the subject is set, such a shape is variable.

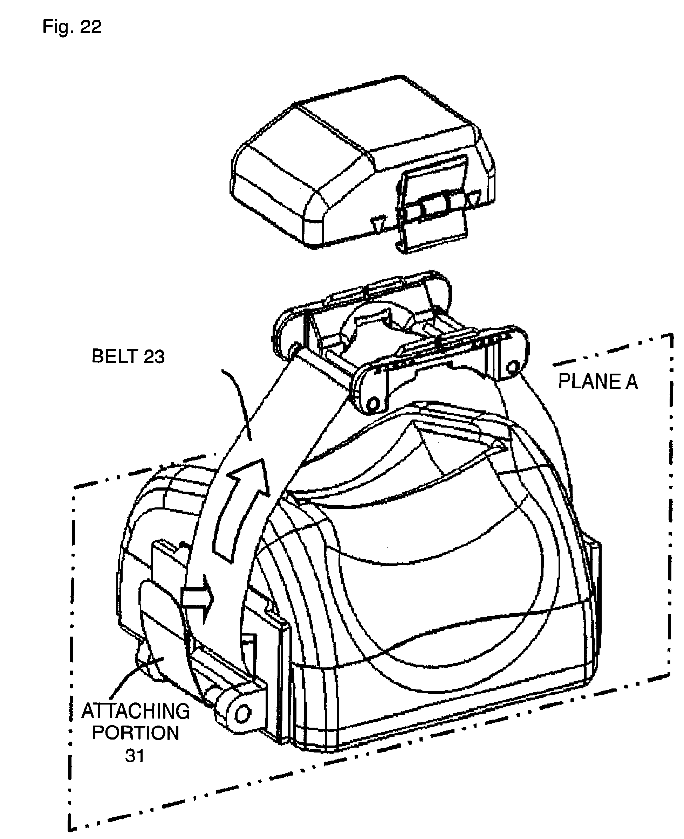

The fixture 3 has an attaching portion 31, and one end of the belt 23 on the base 2 placed in a direction intersecting the artery of the subject is attached to a surface of the fixture 3 parallel with the artery of the subject. Fig. 22 illustrates the attaching portion 31 of the fixture 3.

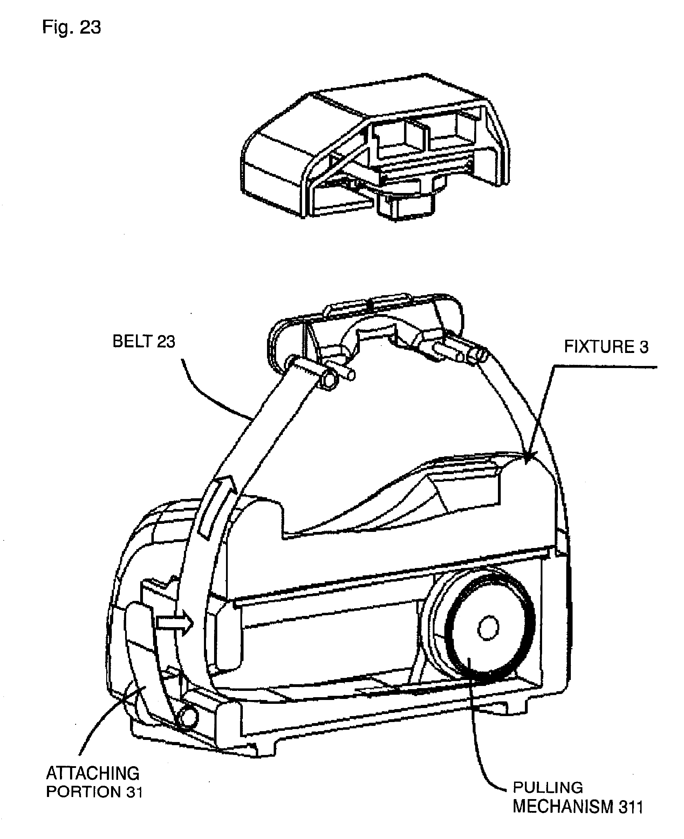

With reference to Fig. 22, the attaching portion 31 does not relatively fix the fixture 3 and the belt 23, and it fixes them so that while one end of the belt 23 is being pulled by a predetermined force, a length of the belt 23 is fixed adjustably within a certain range. More specifically, the measurer pulls the one end of the belt 23 out of the fixture 3 so as to be capable of adjusting its length suitably for the arm of the subject. When a predetermined length is obtained, the belt 23 is set on the attaching portion 31 so as to its tension is fixed. A method of attaching the fixture 3 and one end of the belt 23 to the attaching portion 31 will be described below specifically with reference to Fig. 23. Fig. 23 is a schematic diagram showing a sectional view on a plane A of Fig. 22.

With reference to Fig. 23, the fixture 3 has a pulling mechanism 311 therein, and the pulling mechanism 311 is attached so as to be jointed to one end of the belt 23. The pulling mechanism 311 is a spring, a constant force spring or the like, and when the belt 23 to be jointed is pulled, the pulling mechanism 311 pulls one end of the belt 23 with a predetermined force. When the pulling mechanism 311 is the constant force spring, the pulling mechanism 311 can pull the belt 23 with a predetermined force regardless of a length of the pulled belt 23. The belt 23 can be provided on the attaching portion 31 so that the tension by means of the pulling mechanism 311 is fixed in a position that the belt 23 has a predetermined length. Since the one end of the belt 23 is jointed to the pulling mechanism 311, even if the measurer pulls any portion of the belt 23 other than its end, the belt 23 is pulled out by a predetermined force.

The attaching portion 31 is a surface fastener, a button, a hook, a adhesive tape or the like, and has such a structure as to fix the one end of the belt 23 pulled out from the fixture 3.

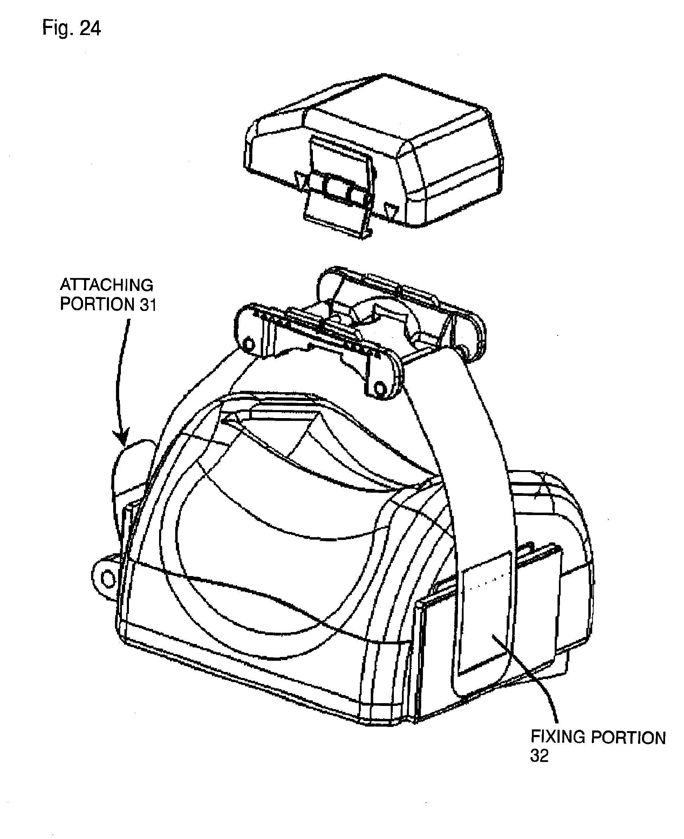

The fixture 3 further has a fixing portion 32 for fixing the other end of the belt 23 to a surface of the fixture 3 opposite to a surface provided with the attaching portion 31. Fig. 24 illustrates the fixing portion 32 of the fixture 3.

With reference to Fig. 24, specifically, the fixing portion 32 of the fixture 3 and the one end of the belt 23 are provided with the surface fastener, the button, the hook, the adhesive tape or the like, so that the length of the belt 23 is set freely, and the fixture 3 and the other end of the belt 23 are fixed.

The measurer pulls the one end of the belt 23 so that the base 2 coincides with a predetermined position of the wrist of the subject so as to fix the one end to the fixture 3 using the fixing portion 32. In order to fix the tension, the measurer sets the other end of the belt 23 to the fixture 3 using the attaching portion 31.

The attaching portion 31 and the fixing portion 32 of the fixture 3 are not limited to the above-described embodiment. That is, the method of fixing on the fixing portion 32 is not limited to the fixing method in which attachment/detachment can be carried out freely. The method may be such that when an optimum position is determined, the surface of the fixture 3 parallel with the artery of the subject and the one end of the belt 23 are fixed completely. The similar case is applied to the setting method on the attaching portion 31.

The fixing portion 32 does not have to employ the detachable structure, and both the attaching portion 31 and the fixing portion 32 which are both ends of the belt 23 may be fixed to the fixture 3. In this case, the both ends of the belt 23 are jointed to the pulling mechanism 311, and both the ends of the belt 23 are pulled by the pulling mechanism 311 with a predetermined force. The both ends of the belt 23 may be held so that its length is adjustable. At this time, when the belt 23 has a predetermined length, the tension may be fixed by the attaching portion provided on the both ends of the belt 23, and when the belt 23 and the base 2 are detachable, the belt 23 with a predetermined length is attached to the base 2 so as that the tension may be fixed. The both ends of the belt 23 are pulled by a predetermined force in such a manner, so that the base 2 is pulled to one direction, thereby preventing the shift from the artery of the subject.

The fixture 3 may be of a cord reel system such that the belt 23 is wound into the fixture 3 by the pulling mechanism 311. Only when the pulse wave monitor measures the pulse wave, the one end of the belt 23 is held so that the belt 23 house in the fixture 3 can be wound out to a predetermined length so as to be used. After the belt 23 is pulled out by controlling the code reel and the first positioning of the base 2 is performed, the belt 23 may be controlled to expand and contract.

When the belt 23 is wound, the position of the base 2 may be automatically determined. Specifically, when the position of the artery is prestored on a memory, not shown, of the fixture 3 with a length of the arm being used as a parameter, the position of the artery of the subject is automatically read from the memory only by pulling out the belt 23 and wounding it around the arm of the subject, so that the base 2 may be automatically set on the artery.

A length of the belt 23 is preadjusted and fixed so that the base 2 is set on the artery of the subject, and the both ends of the belt 2 are jointed to the pulling mechanism 311 and pulled. As a result, the measurer can always set the base 2 on the artery position of the subject automatically without palpating to detect the artery position every time of the measurement of the pulse wave.

The length of the belt 23 is preadjusted so that the base 2 is mounted on the artery of the subject, and the belt 23 is fixed to attached to the attaching portion 31 completely. As a result, at the time of the measurement thereinafter, the base 2 can be always mounted on the artery of the subject automatically.

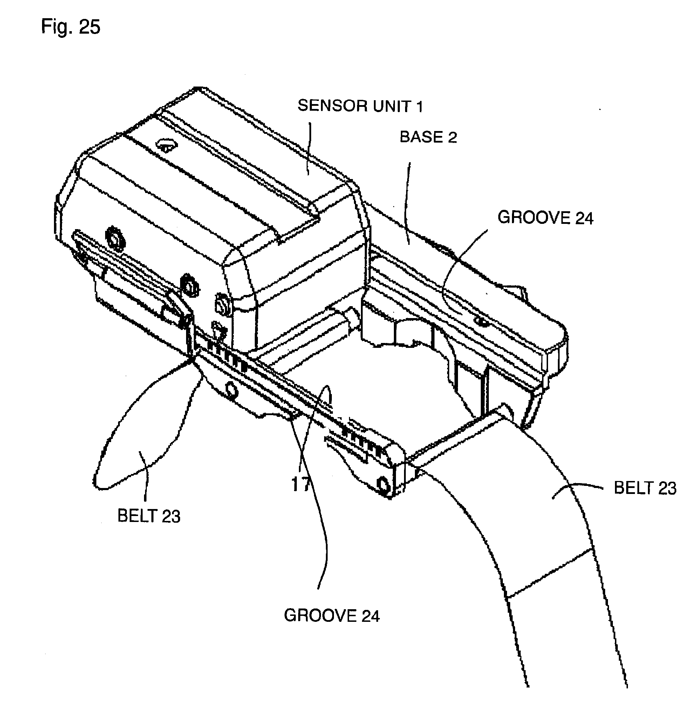

The description has been given as to the pulse wave monitor of the above embodiment in which the sensor unit 1 and the base 2 are separated units. As shown in Fig. 25, however, the pulse wave monitor may be structured so that the sensor unit 1 and the base unit 2 are integrated as one unit. The sensor unit 1 is formed of a box-shaped member having an opening on its lower surface, and has a sensor 11 (not shown) therein. The belt 23 is attached to a predetermined position of the base 2 as described above. The base 2 is a member for fixing the sensor unit 1 to an organism. When the sensor unit 1 and the base 2 are integrated as one unit in such a manner, the pulse wave sensor 111 can be easily set on a position of a body surface to be pressured by the sensor 11 (that is, the position of the body surface just on the artery). That is, after a user checks the position of the body surface to be pressured by the sensor 11 by means of palpation or the like, the base 2 is fixed to the organism of the subject so that the position comes to the center of the base 2, and the sensor unit 1 is slid along the base 2 to be fixed, thereby mounting the sensor 11 just on the artery simply.

In the pulse wave monitor shown in Fig. 25, the sensor unit 1 is incorporated into the base 2 slidably. Specifically, the sensor unit 1 is incorporated into the base 2 so as to be capable of sliding along the grooves 24 formed on the base 2. At the time when the base 2 is attached to the portion of the subject through which the pulse wave is measured by the belt 23, the sensor unit 1 is retreated to one end of the grooves 24 of the base 2 (left end in the figure), and the first positioning is performed. Thereafter, the measurer slides the sensor unit 1 along the grooves 24 so as to be capable of mounting the sensor unit 1 approximately just on the artery of the subject. The sensor unit 1 is incorporated into the base 2 slidably, thereby realizing the pulse wave monitor having excellent operability and the simple structure.

### (8) Use of Pulse Wave Monitor According to Embodiment

When the pulse wave monitor according to the embodiment is used, the measurer can easily position the sensor unit 1. That is, as described above, the pulse wave monitor of the embodiment is characterized by performing the two-stage positioning. In the first positioning, while the pulse wave of the subject is being palpated to be detected through the pulse detecting hole 22, the base 2 can be mounted. Therefore, since the first positioning can be performed without detaching a finger, the pulse wave detector or the like from the subject, the accuracy of the first positioning is improved greatly, so that the sensor unit 1 can be set approximately just on the artery of the subject in the first positioning. In the case where while the artery of the subject is being detected through the pulse detecting hole 22 by the pulse wave detector or the like such as a Doppler blood-flowmetry, the first positioning is performed, the first positioning can be performed more accurately than the palpation. The convenience of the measurer can be therefore improved.

Since the through pulse detecting hole 22 is provided on the base 2 of the pulse wave monitor of the embodiment, the finger, the pulse wave detector or the like can be directly brought into contact with the subject, the detection of the artery becomes easy, thereby improving the accuracy of the first positioning.

When the base 2 of the pulse wave monitor of the embodiment is provided with the pulse detecting hole 22 on which the film, thickness and material of which are such that the detection of the artery of the subject is not hindered, is laid, the sensor surface of the sensor unit 1 can be protected.

In the pulse wave monitor according to the embodiment, since the sensor unit 1 can be moved to a arbitrary position of the base 2 and fixed, even if the position of the sensor unit 1 shifts from the artery in the first positioning, it is not necessary to again set the base 2 on the subject, and only the positioning of the sensor unit 1 (the second positioning) is performed, so that the pulse wave sensor 111 of the sensor unit 1 can be arranged on the artery of the subject accurately and securely. Time and troublesomeness required for the measurement of the pulse wave can be therefore reduced greatly, thereby reducing a load on the measurer and the subject greatly.

In the pulse wave monitor of the embodiment, since the sensor unit 1 can be moved and fixed manually, the positioning can be performed without a cost. At this time, the mark, which are marked in a direction crossing the artery of the subject, are provided on the joint surface between the sensor unit 1 and the base 2, and this mark become the guidepost of a moving amount of the sensor unit 1 at the time of performing the second positioning, thereby improving the convenience of the measurer.

Since the pulse wave monitor according to the embodiment does not have to be mounted with a motor or the like for moving and fixing the sensor unit 1, miniaturization and lightening of the pulse wave monitor can be realized.

Since the fixture 3 is provided on the pulse wave monitor according to the embodiment, the pulse wave can be measured in a state that the base 2 is stably set on the wrist of the subject, thereby measuring the pulse wave accurately.

The base 2 is pulled from the fixture 3 by a constant force via the belt 23, so that the belt 23 is wound around the subject with a suitable force. As a result, the base 2 is stably fixed to the subject, and if the subject moves the arm, the base 2 is prevented from shifting from the subject. Surplus fastening does not occur, so that the subject is not hurt.

While palpating the subject with one hand, the measurer can perform the positioning with the other hand, thereby improving the convenience of the measurer greatly. On the contrary, the fixture 3 can be fixed with a subject's hand, and the measurer can perform the positioning with the other hand. A measurer other than the subject is not, therefore, required, the subject can measure the pulse wave by himself (herself), thereby helping realization of self-medication.

Needless to say, the pulse wave monitor according to the embodiment of the present invention has a plurality of the pulse wave sensors 111 for compensating adjustment due to the second positioning on the sensor 111 of the sensor unit 1, the second positioning is not occasionally performed. That is, in this case, when the base 2 is mounted on the artery of the subject and the sensor unit 1 is mounted on the base 2, at least one of the pulse wave sensors 111 provided on the sensor unit 1 is set just on the artery of the subject. The second positioning, therefore, is not necessary, and the sensor unit 1 does not have to be slid.

When only the palpation and the artery detector can set the base 2 just on the artery of the subject with sufficient accuracy, the second positioning can be eliminated.

The embodiment disclosed here should be considered to be only the example in all points and is not limitative. The scope of the present invention is defined not by the above description but by following claims, and the invention is intended to include all modifications within the means and the scope equivalent to the scope of the invention.

## Claims

1. A pulse wave monitor measuring a pulse wave of a subject, comprising:
a sensor unit comprising a sensor detecting the pulse wave;
a base configured to be mounted on a portion of the subject and to engage with the sensor unit, the base comprising a positioning portion through which an artery of the subject is detected; and
a slide mechanism for sliding the sensor unit relative to the base when the base is engaged with the sensor unit.

2. The pulse wave monitor of claim 1, wherein the positioning portion of the base has an opening through which the artery of the subject is detected.

3. The pulse wave monitor of claim 1, wherein the positioning portion of the base has a hole through which the artery of the subject is detected.

4. The pulse wave monitor of claim 1, wherein the positioning portion of the base has a cut away portion through which the artery of the subject is detected.

5. The pulse wave monitor of claim 1, further comprising a display providing information about a positioning of the sensor with respect to the artery.

6. The pulse wave monitor of claim 1, wherein an array of markings are formed on the sensor unit, the base or the slide mechanism so that the array is positioned perpendicular to the direction of the artery when the base is mounted on the portion of the subject and is engaged with the sensor unit.

7. The pulse wave monitor of claim 1, further comprising a subject fixture comprising a first attaching portion and a second attaching portion, wherein a first portion of the base is attached to the first attaching portion and a second portion of the base is adjustably attached to the second attaching portion.

8. The pulse wave monitor of claim 7, wherein the subject fixture further comprising a pulling mechanism that applies a tension to the second portion of the base.

9. A pulse wave monitor measuring a pulse wave of a subject, comprising:
a base configured to be mounted on a portion of the subject; a sensor unit comprising a sensor detecting the pulse wave; means for coarsely positioning the base on the portion of the subject;
means for finely positioning the sensor unit with respect to the portion of the subject.

10. The pulse wave monitor of claim 9, further comprising a subject fixture comprising a first attaching portion and a second attaching portion, wherein a first portion of the base is attached to the first attaching portion and a second portion of the base is adjustably attached to the second attaching portion.

11. The pulse wave monitor of claim 9, wherein the subject fixture further comprising a pulling mechanism that applies a tension to the second portion of the base.
